(19)

Europäisches
Patentamt

European
Patent Office

Office européen
des brevets

(11)  **EP 2 884 284 A1**

(12)  **EUROPEAN PATENT APPLICATION**

(43) Date of publication:
**17.06.2015  Bulletin 2015/25**

(51) Int Cl.:
***G01N 33/86*** [(2006.01)]

(21) Application number: **13196975.0**

(22) Date of filing: **12.12.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
• **C A Casyso AG**
  **4132 Muttenz (CH)**
• **Friedrich-Alexander-Universität Erlangen-Nürnberg**
  **91054 Erlangen (DE)**

(72) Inventors:
• **Erber, Matthias**
  **91054 Erlangen (DE)**
• **Lee, Geoffrey**
  **91080 Uttenreuth (DE)**
• **Schwaiger, Martin**
  **81667 München (DE)**
• **Schubert, Axel**
  **81927 München (DE)**

(74) Representative: **Isarpatent**
  **Patent- und Rechtsanwälte**
  **Friedrichstrasse 31**
  **80801 München (DE)**

(54)  **Dry diagnostic reagent**

(57)    The present invention relates to a dry diagnostic reagent comprising at least a highly water soluble and low hygroscopic compound resulting from a mixture of two or more organic $Ca^{2+}$ salts that interact on the molecular level in a manner that at least partially prevents crystallization of the resulting compound and the use of such a reagent in an in vitro method for measuring the coagulation of a blood sample.

EP 2 884 284 A1

**Description**

The present invention relates to a dry diagnostic reagent, and in particular to a dry diagnostic reagent comprising at least a highly water soluble and low hygroscopic compound resulting from a mixture of two or more organic $Ca^{2+}$ salts that interact on the molecular level in a manner that at least partially prevents crystallization of the compound, which can be suitable for in vitro blood coagulation measurement.

**Background of the invention**

Many coagulation factors involved in blood coagulation need contact to a specifically composed membrane to express their whole catalytic activity. Calcium ions ($Ca^{2+}$) present in blood connect the negatively - charged membrane surface which contains mostly phosphatidylcholine and phosphatidylserine with negatively - charged carbonyl groups of the coagulation factors. Therefore calcium ions circulating in blood are an essential part of the coagulation process and it is possible to inhibit blood coagulation completely by removing calcium from a sample. For this purpose substances which are able to form a stable complex with $Ca^{2+}$ are added to a blood sample, e.g. Na-EDTA, citrate. Blood has been taken according to this principle for decades, because of the possibility to restore complete coagulation activity by adding calcium ions to the sample.

Today there are different in - vitro diagnostic techniques available commercially on the market using whole blood. The blood sample must be recalcified up to a specific threshold (Ataullakhanov, F.I., et al., Calcium threshold in human plasma clotting kinetics, Thrombosis Research, 1994. 75(4): p. 383-394; James, M.F.M. and A.M. Roche, Dose-response relationship between plasma ionized calcium concentration and thrombelastography, Journal of Cardiothoracic and Vascular Anesthesia, 2004, 18(5): p. 581-586) before measurement in order to restore blood coagulation which is commonly achieved by adding a solution containing $CaCl_2$ which has a high solubility in water (~700g/L).

However, it is difficult to measure the clotting properties of whole blood in vitro without influencing the system, due to addition of citrate or protein and cell adsorption to surfaces and dilution due to recalcification (Rand, M.D., et al., Blood clotting in minimally altered whole blood. Blood, 1996, 88(9): p. 3432; Roche, A.M., et al., Citrated Blood Does Not Reliably Reflect Fresh Whole Blood Coagulability in Trials of In Vitro Hemodilution, Anesthesia & Analgesia, 2003. 96(1): p. 58-61). The use of dry, solid reagents for whole blood (WB) coagulation therefore provides a clear advantage over liquid reagents because there is no additional dilution during recalcification. In addition, a solid reagent for recalcification has another advantage over liquid reagents: solid reagents have no need for pipettes and are quality controlled having a constant mass and concentration, so the user only has to add whole blood to the reagent without many pipetting steps before measuring. This reduces the possibilities of errors due to wrong handling and provides the user a ready - to - use diagnostic tool.

$CaCl_2$ is, however, not suitable for the production of a solid reagent because in the solid state $CaCl_2$ is extremely hygroscopic, thus potentially leading to a dilution of a sample. In some cases it will turn to liquid, i.e. gets dissolved, where water absorption depends on temperature and air humidity.

Organic calcium salts, taken alone, are on the other hand mostly not hygroscopic but do not readily dissolve in water or blood. For example calcium lactate dissolves at 66 g/L in water, whereas calcium gluconate dissolves at 35 g/L in water.

There is thus a need for diagnostic reagents that are suitable for overcoming the problems of the state of the art.

**Summary of the invention**

The present inventors found out that it is possible to overcome the above problems by the dry diagnostic reagent according to claim 1. Further provided in the present invention are a diagnostic reagent or kit-of parts comprising the present diagnostic reagent, the use of the present diagnostic reagent for recalcification in *in vitro* coagulation measurements of blood sample and an in vitro method for measuring the coagulation of a blood sample in which the present diagnostic reagent is applied.

Preferred embodiments of the present invention are disclosed in dependent claims and the following description.

**Description of Figures**

The invention will be now described in detail with reference to exemplary embodiments in the accompanying Figures. While the invention will be disclosed in more detail with reference to these Figures, the Figures are not intended to limit the scope of the invention.

In the Figures the following is disclosed:

Fig. 1 shows the mass increase as well as the relative humidity measured in Example 1.

Figure 2 shows the results of the ROTEM® measurement in Example 2.

Figure 3 shows comparative results on contact pathway activation of whole blood in Example 2.

Figure 4 shows X-ray powder diffraction diagrams of the educts in comparison to the produced compound (Figure 4a) as well as freeze-dried compound (Figure 4b) obtained as described in Example 3.

Figure 5 shows the results of a differential scanning calorimetry analysis of a sample prepared in Example 3.

Figure 6 shows a SEM picture of a sample prepared in Example 3.

Figure 7 shows results of a comparison between different amounts of reagent added to recalcify a whole blood sample as obtained in Example 4.

Figure 8 (a) shows X-ray powder diffraction diagrams obtained in Example 5. Figure 8 (b) to (g) show the single results for the respective substances obtained in Example 5.

Figure 9 (a) to (c) shows the results of hygroscopicity measurements conducted in Example 5.

Figure 10 shows the results of a recalcification experiment in Example 5.

Figure 11 shows the results of hygroscopicity measurements in Example 6.

Figure 12 (a) to (c) show the dependency of the glass transition on the composition of the substance and on residual moisture content of exemplary compounds in the Examples.

Figure 13 shows a Differential Scanning Calorimeter curve demonstrating the maximally frozen system's glass transition temperature of a solution in Example 5.

Figure 14 shows the result of an X-ray powder diffraction of CL produced with the rotary evaporation method described below demonstrating CL crystallinity.

**Detailed description of the invention**

Definitions:

A diagnostic reagent in the present invention is considered "dry" if it contains less than 1% by weight of solvent, preferably water, based on the total weight of the diagnostic reagent, preferably less than 0.5% by weight, more preferably less than 0.1% by weight, further more preferably less than 0.01% by weight and particularly does not contain any solvent at all, at least no water.

Within this invention, a compound resulting from a mixture of two or more organic $Ca^{2+}$ salts is highly water soluble if it can be easily dissolved in water at ambient temperature (about 20°C, e.g. about 15 - 30°C or 20 - 26°C), particularly at about 20 - 25°C, e.g. about 20°C, and ambient pressure (around about 101.325 kPa, e.g. between 90 and 105 kPa), particularly at about 101.325 kPa at the normal pH of water of about 7.0. Preferable is a solubility in water of at least 100 g/l, preferably at least 200 g/l, further preferably at least 250 g/l, more preferably at least 300 g/l and particularly preferably of at least 400g/l, particularly at about 20°C and about 101.325 kPa. Preferable is a solubility of the present compound with regard to calcium ions ($Ca^{2+}$) of 0.8 mol/L, preferably 1.0 mol/l and further preferably 1.1 mol/l, particularly preferably 1.2 mol/l, particularly at about 20°C and about 101.325 kPa.

Further, within the present invention, a compound resulting from a mixture of two or more organic $Ca^{2+}$ salts has low hygroscopicity if it does not attract and/or hold water molecules from the surrounding environment at ambient surroundings at a relative humidity of air between 10 and 100% to a big extent. Particularly, the compound in the present invention is less hygroscopic than $CaCl_2$. Preferably the hygroscopicity of the compound resulting from a mixture of two or more organic $Ca^{2+}$ salts as measured by mass increase at relative air humidity >50% is <5% mass over 96 hours, preferably <1% over 96 hours, and more preferably <0.1% over 96 hours.

A compound resulting from a mixture of two or more organic $Ca^{2+}$ salts that interact on the molecular level is a compound that contains two or more components that interact with each other, i.e. wherein the two or more individual compounds that are forming the mixture cannot be found as separate phases therein anymore when analyzing the compound, e.g. by X-ray diffraction. Upon dissolution of the two components in a solvent like water the two or more

organic $Ca^{2+}$ salts can be again found as analytes within the obtained solution.

The two or more organic $Ca^{2+}$ salts interact on a molecular level in a manner that at least partially prevents crystallization if not the whole combined mass of the two or more organic $Ca^{2+}$ salts in the resulting compound is present in crystallized form after mixing the salts. In certain embodiments a chemical reaction wherein covalent bonds are formed does not take place, as can be confirmed by e.g. 1H NMR using $D_2O$ wherein the components can be observed individually. Preferably at least 50 percent of the total mass of the salts is not present in crystallized form, further preferably at least 75 %, even further preferably at least 90 %, more preferably at least 95 % and particularly preferably at least 99 % of the total mass of the salts is not present in crystallized form. In preferred embodiments the resulting compound of the mixing of the two or more organic $Ca^{2+}$ salts is present in amorphous form, preferably as solid, i.e. is present in a form that lacks the long-range order of a crystal. Thus preferably crystallization is avoided. An amorphous solid can be seen as a solution of the components in each other that are present as solid due to increased viscosity.

In the present invention a stereoisomer of a compound is a compound that has the same structural formula and configuration, i.e. is not a constitutional (structural isomer), but the geometrical positioning of bonds and functional groups/atoms differs. Encompassed therein are enantiomers, diastereomers, conformers, rotamers, cis/trans isomers.

A fruit acid in terms of the present invention is a collective term that encompasses organic acids that frequently are encountered in fruit. Encompassed are particularly hydroxycarboxylic acids like citric acid, lactic acid, malic acid, tartaric acid, gluconic acid, mandelic acid, $\alpha$-hydroxy caprylic acid, glycolic acid and dicarboxylic acids like fumaric acid, oxalic acid, saccharic acid or succinic acid as well as salicylic acid (Römpp Chemie Lexikon Cm - G, 9th ed., 1990, p. 1448 - 1449; http://de.wikipedia.org/wiki/Fruchts%C3%A4uren).

A fatty acid within the present invention is a carboxylic acid having 4 to 40 carbon atoms, preferably 4 to 20 carbon atoms, further preferably 8 to 20 carbon atoms, with an aliphatic tail (chain), which is either saturated or unsaturated. Fatty acids with a lower carbon atom number are preferable as they can be prepared easier due to a better solubility in water.

Further, a blood sample in the present invention can comprise whole blood, i.e. the blood from one donor which may or may not contain an anticoagulant which can be added after taking a blood sample, plasma/blood plasma, i.e. the liquid component of blood without the cellular components, etc.

In the present invention, the glass transition is the transition in amorphous materials from a hard and relatively brittle state into a molten or rubber-like state. The glass transition temperature Tg is the temperature where glass transition occurs. Further, the temperature Tg' is the glass transition temperature of the maximally frozen system, i.e. the glass transition of the amorphous phase within an ice matrix (from ultrapure water). This temperature is closely connected with the collapse temperature Tc. Although these two temperatures are not identical, Tg' determination using DSC (Differential Scanning Calorimetry) provides a good approach to solution stability, especially during primary drying in a freeze drying process. The collapse temperature itself is the temperature above which collapse of the porous cake structure during primary drying in the region of the ice - water interface occurs due to increased mobility of the amorphous phase. An increase in shelf temperature of about 1°C may result in a ~13% reduction of primary drying time [Pikal, M. J. and S. Shah (1990). "The collapse temperature in freeze drying: Dependence on measurement methodology and rate of water removal from the glassy phase." International Journal of Pharmaceutics 62(2-3): 165-186]. Since Tc defines the maximum process temperature during primary drying ($T_{shelf}$ ~ Tc - 5°C; resulting in a product temperature close to Tc) this temperature and therefore also Tg' are of paramount importance for a highly economical process. Considering these points Tg' of the compound lies preferably above -30°C (243K), further preferably above -20°C (253K) and most preferably above -15°C (258K).

The present invention relates to a dry diagnostic reagent comprising at least a highly water soluble and low hygroscopic compound resulting from a mixture of two or more organic $Ca^{2+}$ salts that interact on the molecular level in a manner that at least partially prevents crystallization of the resulting compound (hereinafter also termed "resulting compound").

While two or more organic $Ca^{2+}$ salts can be comprised in the mixture of the present dry diagnostic reagent, preferably two to four, further preferably two to three and particularly preferably two organic $Ca^{2+}$ salts are comprised in the mixture. While two to four organic $Ca^{2+}$ salts will enable a better formation of the resulting compound, a further improved formation of the resulting compound will be achieved with two or three organic $Ca^{2+}$ salts. The easiest and most stable formation of the resulting compound is achieved when two organic $Ca^{2+}$ salts are used.

In preferred embodiments, at least one salt of the two or more organic $Ca^{2+}$ salts is formed by an organic acid, preferably a saturated or non-saturated carboxylic acid, preferably an $\alpha$-, $\beta$-, or y-hydroxy acid, a polyhydroxy acid, a dicarboxylic acid, a tricarboxylic acid, a phenolic carboxylic acid, a cyclic carboxylic acid or a heterocyclic carboxylic acid. Particularly preferred carboxylic acids are fruit acids, preferably malic acid, mandelic acid, tartaric acid, $\alpha$-hydroxy caprylic acid, oxalic acid, glycolic acid, lactic acid, citric acid, salicylic acid, gluconic acid, saccharic acid, fumaric acid, or stereoisomers thereof. Other particularly preferred carboxylic acids are fatty acids, e.g. caprylic acid, capric acid, lauric acid, myristic acid, palmitic acid, arachidic acid, behenic acid, palmitoleic acid, elaidic acid, linoleic acid, linoelaidic acid, $\alpha$-linoleic acid, erucic acid, eicosapentaenoic acid, stearic acid, oleic acid, arachidonic acid, or stereoisomers thereof. The organic acid preferably contains 1 to 40 C-atoms, further preferably 1 to 30 C-atoms, even further preferably 1 - 25 C-atoms, more preferably 1 -20 C-atoms and particularly preferably 1 - 10 C-atoms.

It is preferred that the present diagnostic reagent contains at least two calcium salts of two different carboxylic acids, e.g. independently selected from calcium lactate, calcium gluconate, calcium saccharate, calcium lactobionate, calcium ascorbate, calcium pantothenate or calcium glycolate, or a combination of a fruit acid and a fatty acid.

In case two calcium salts are used, it is preferred that a molar ratio of the two salts in the mixture is 0.1 - 2 : 1, further preferably 0.5 - 1.5 : 1. In case three or more calcium salts are used, it is preferred that each calcium salts except for one reference calcium salt in the mixture are used in a molar ratio of 0.1 - 2 (other calcium salt) : 1 (reference calcium salt), further preferably 0.5 - 1.5 (other calcium salt) : 1 (reference calcium salt). For example, calcium lactate and calcium gluconate, calcium lactate and calcium lactobionate, calcium lactate and calcium ascorbate, calcium ascorbate and calcium lactobionate, calcium lactobionate and calcium pantothenate, calcium ascorbate and calcium gluconate, or calcium lactate and calcium pantothenate can be preferably applied at a ratio of 0.1 - 2 : 1, further preferably 0.5 - 1.5 : 1 in the mixture.

In particularly preferred embodiments the two calcium salts are present in an approximately equimolar ratio (within a range of 0.95 - 1.05 : 1, preferably 0.99 - 1.01 : 1, more preferably 1 : 1) and preferably are calcium lacto gluconate, calcium lacto lactobionate, calcium lacto ascorbate and calcium lacto pantothenate, calcium ascorbate lactobionate, calcium lactobionate pantothenate, calcium ascorbate gluconate, further preferably calcium lacto gluconate. It is also possible that a mixture of two or more of these compounds is present. It is preferable that the two or more calcium salts have a low hygroscopicity, as measured by mass increase at relative air humidity >50%, of <5% mass over 96 hours.

Preferred resulting compounds of the mixtures of the two or more organic $Ca^{2+}$ salts are thus calcium lacto gluconate (CLG), calcium lacto lactobionate (CLLB), calcium lacto ascorbate (CLA) and calcium lacto panthothenate (CLP), calcium ascorbate lactobionate (CALB), calcium lactobionate pantothenate (CLBP), calcium ascorbate gluconate (CAG), and a particularly preferred compound resulting from mixing of the two or more organic $Ca^{2+}$ salts is calcium lactate gluconate which shows surprisingly good results in coagulation experiments.

In further preferred embodiments the compound resulting from a mixture of two or more organic $Ca^{2+}$ salts that interact on the molecular level in a manner that at least partially prevents crystallization of the resulting compound has a glass transition temperature Tg of at least 55°C, preferably at least 75°C, further preferably at least 80°C and particularly preferably at least 85°C. Even more preferably the glass transition temperature is higher than 90°C so that storage at 40°C, i.e. even under extreme temperature conditions, is possible. The higher the glass transition temperature is, the better the stability of the compound, which is generally metastable.

In certain embodiments the compound resulting from a mixture of two or more organic $Ca^{2+}$ salts that interact on the molecular level in a manner that at least partially prevents crystallization of the resulting compound further has a dissolution time of less than 60 s, preferably less than 45 s and particularly less than 30 seconds in water at a temperature of about 20 - 25°C, e.g. about 20°C, and ambient pressure.

Apart from the two or more organic $Ca^{2+}$ salts the present dry diagnostic reagent can further comprise an inorganic $Ca^{2+}$ salt like $CaCl_2$, preferably $CaCl_2$. In certain embodiments a preferred combination of salts is thus calcium lacto gluconate, calcium lacto lactobionate, calcium lacto ascorbate and calcium lacto pantothenate, calcium ascorbate lactobionate, calcium lactobionate pantothenate, and/or calcium ascorbate gluconate with $CaCl_2$, e.g. calcium lacto gluconate with $CaCl_2$, which achieves a lower molar mass and is less hygroscopic then $CaCl_2$. In certain embodiments it is possible to provide the dry diagnostic reagent in a form wherein $CaCl_2$ is coated with a highly water soluble and low hygroscopic compound resulting from a mixture of two or more organic $Ca^{2+}$ salts that interact on the molecular level in a manner that at least partially prevents crystallization of the resulting compound, e.g. calcium lacto gluconate, calcium lacto lactobionate, calcium lacto ascorbate and calcium lacto pantothenate, calcium ascorbate lactobionate, calcium lactobionate pantothenate, and/or calcium ascorbate gluconate.

The present dry diagnostic reagent is preferably used in solid form, and particularly preferably used in lyophilized form. The production of the lyophilized diagnostic reagent can be carried out with any known method, e.g. freeze-drying using a manifold freeze-dryer, a rotary freeze-dryer or a tray style freeze-dryer. It can be carried out by spray-drying, vacuum-drying or other known methods used for freeze-drying. A freeze-dried sample shows improved dissolution in a blood sample, thus leading to faster results and avoiding problems related to e.g. clotting in coagulation experiments.

As a solid source of calcium, the present dry diagnostic reagent can be used for in vitro coagulation measurement of a blood sample, e.g. whole blood, blood plasma etc., i.e. activated partial Thromboplastin time (aPTT), Prothrombin time (PT); Thromboelastometry (ROTEM); Thromboelastography (TEG). Furthermore, the present diagnostic reagent can also be used for recalcification in in vitro coagulation measurements of a blood sample, e.g. whole blood, blood plasma etc. In certain embodiments the blood sample is selected from whole blood or blood plasma and was prepared to prevent clotting activity by $Ca^{2+}$ complexing or $Ca^{2+}$ removal. In such embodiments, $Ca^{2+}$ complexing was achieved by using sample tubes containing a $Ca^{2+}$ complexing agent, e.g. sodium citrate or sodium EDTA. Particular useful for these applications is calcium lacto gluconate.

It is especially surprising in the present invention that the organic residue, i.e. the organic acid part, of the organic $Ca^{2+}$ salts does not significantly influence/disturb the coagulation measurement as measured by coagulation diagnostics, e.g. the clotting time, the clot formation time, the clot amplitude or other coagulation parameters, i.e. that the anions do not negatively affect the parameters in the methods for determining blood coagulation. Particularly surprising is that even anions like a lactate residue do not interfere with the measurement.

The present invention further provides a diagnostic composition or kit-of-parts comprising the present diagnostic reagent. A diagnostic composition can thereby in certain embodiments comprise further compounds that are useful in the above applications, e.g. anticoagulants like sodium citrate or sodium EDTA, further coagulation activators (e.g. tissue factor, ellagic acid, kaolin, ecarin, etc.), coagulation inhibitors (e.g. Heparin, Hirudin, Factor IIa - Inhibitors, Factor Xa - Inhibitors), pH adjusting agents (e.g. hydrochloric acid, sodium hydroxide), organic and/or inorganic buffers (e.g. HEPES, TRIS, potassium phosphate, sodium carbonate, etc.), etc. In a kit-of-parts further components can be contained in addition to the present dry diagnostic reagent, like a test tube, a syringe for drawing blood, a cover for the syringe, a band-aid, a disinfectant, a cotton ball, and other useful components that can be applied in the above applications. The kit-of-parts also can contain the present diagnostic composition in place of the dry diagnostic reagent.

Further disclosed within the present invention is an in vitro method for measuring the coagulation of a blood sample comprising

a) providing a blood sample from a patient,
b) complexing $Ca^{2+}$ in the blood sample by a complexing agent, preferably sodium EDTA or sodium citrate,
c) recalcifying the blood sample by adding a suitable amount of the present diagnostic reagent,
d) optionally adding further coagulation activators, coagulation inhibitors, or other substances, and
d) performing the coagulation measurement.

In certain embodiments of the present in vitro method the recalcification of the blood sample in step c) is carried out without changing the coagulation properties of the sample / blood sample.

A suitable amount of the present diagnostic reagent in step c) can thereby be suitably determined based on the amount of sample taken and the type of blood sample. For example, a suitable amount for one milliliter of whole blood would be ~ 11.76 μmol, i.e. 3.95mg, for a 1:1 mixture of calcium lacto gluconate.

In certain embodiments, the coagulation measurement includes the activated partial thromboplastin time (aPTT), prothrombin time (PT), and/or viscoelastic tests like thromboelastometry/thromboelastography. These measurements are known to the skilled person from e.g. EP 2 202 517 A1, EP 2 553 470, EP 0 972 203.

In this regard it is surprising that the anions of the organic $Ca^{2+}$ salts that interact on the molecular level and are present in the present dry diagnostic reagent do not significantly influence the normal coagulation behavior of a blood sample / plasma sample as measured by coagulation diagnostics, e.g. the clotting time, the clot formation time, the clot amplitude or other coagulation parameters.

The invention will in the following be described in more detail with reference to specific examples thereof. While the invention is described with reference to specific examples thereof, it is clear to the skilled person that the scope of the invention is not limited to these examples.

**Examples**

Example 1: Hygroscopicity measurements

Calcium lacto gluconate (CLG) was produced by combining an aqueous solution of calcium lactate (CL), in the form of a hydrate, calcium lactate x 1.5 $H_2O$, and an aqueous solution of calcium gluconate (CG), in form of a hydrate, calcium gluconate x $H_2O$, each in ultrapure water (demineralized, double-distilled water that was aseptically filtrated with a 0.23 μm filter prior to usage) at a concentration of 0.06 mol/L (solubility of CL in water: 0.36 mol/L; solubility of CG in water: 0.07 mol/L), generating a solution where the single excipients are concentrated at equimolar ratio, i.e. at a molar ratio of 1:1. Afterwards the solution is transferred to a rotary evaporator (Heidolph VV2000 connected to a Vacuumbrand CVC3000 vacuum pump; the temperature was adjusted using a Heidolph WB 2000 water bath) where the water is removed.

Settings:

T: 80 °C
Pressure: 30 mbar
Rotation speed: 120 rpm
Drying time: 30 min

As a reference, $CaCl_2$ powder is used.

During the experiment 1.0g CLG and 1.0g $CaCl_2$ were placed in a glove box where the relative air humidity was > 50% which was measured using a testo 605-H1 "mini humidity stick" dew point hygrometer. The temperature in the glove box was uncontrolled between 22.5°C and 25.6°C during this experiment.

The hygroscopicity of compounds was measured by weighing (Mettler AT261 "Delta Range" balance) in the samples at the start of the experiment and at certain time points within a total period of 96 hours.
The increase in mass (in %) was calculated for each sample as:

$$\frac{m_t - m_{t0}}{m_{t0}} \times 100\%$$

wherein $m_t$ is the mass of the sample at a certain time t and $M_{t0}$ is the mass of the sample at the start when placing it in the glove box.

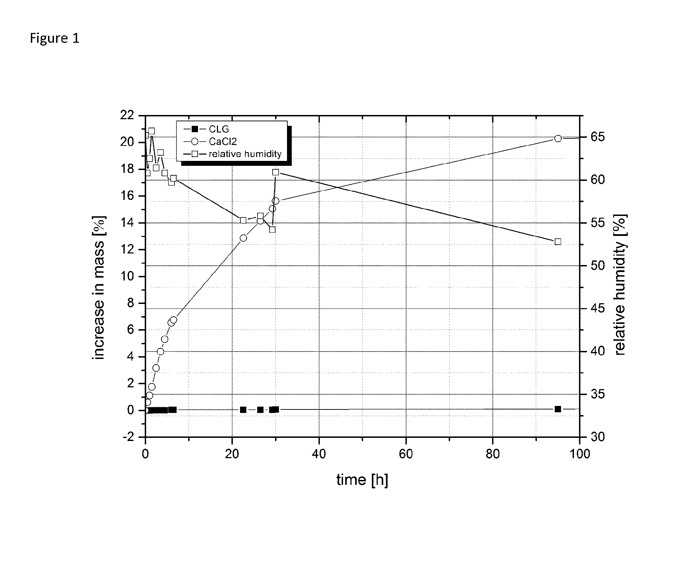

The results for the measurements of mass increase of CLG and $CaCl_2$ as well as the measurements for the relative humidity (in %) of the experiment are shown in Figure 1.

Surprisingly it has been found that CLG can be prepared in the solid state and has much lower hygroscopicity than $CaCl_2$. As seen from Figure 1, CaCl2 increased its mass over 20% referring to the initial mass and was clearly deliquescent. The mass increase of CLG was <0.1%.

The resulting CLG solid further has a high solubility in water (~400g/L), as determined by dissolving the product in ultrapure water, and shows very low hygroscopicity compared to $CaCl_2$, which gives it a clear advantage over $CaCl_2$ regarding solid products and handling conditions. Using CLG as a source for $Ca^{2+}$ in a solid dosage form will avoid high moisture uptake and therefore rising moisture content up to fluidization in the final product over time due to hygroscopicity. In addition the high solubility of CLG in water provides the possibility to generate highly concentrated solutions or to generate a high $Ca^{2+}$ concentration in a blood sample without adding much solid.

For further experiments, solutions of the produced CLG at concentrations of 320 mg/mL and 396 mg/mL were prepared by dissolving appropriate amounts of the CLG in ultrapure water. Also, for further testing in the further examples the samples were stored in a desiccator over a saturated $K_2SO_4$ solution at a relative humidity of about 100%. The dessicator was placed in a climate chamber at 25°C

Example 2: Comparison of CLG to standard reagent in coagulation and contact pathway activation

The influence of CLG on coagulation was determined using a ROTEM® device (TEM Innovations GmbH, Germany) and whole blood from healthy volunteers using either the EXTEM assay or the NATEM assay. Coagulation was therefore either initiated using the tissue factor based ex-tem® reagent (+recalcification) (TEM Innovations GmbH, Germany) or only by recalcification of the citrated whole blood sample. During the experiment CLG solutions with concentrations of 320 mg/mL and 396 mg/mL were compared to a star-tem@ reagent (0.2 M $CaCl_2$ solution; ROTEM® reagent; TEM Innovations GmbH, Germany). Solutions of CLG were taken to show the influence of the substance independent of the dissolution behavior of the substance.

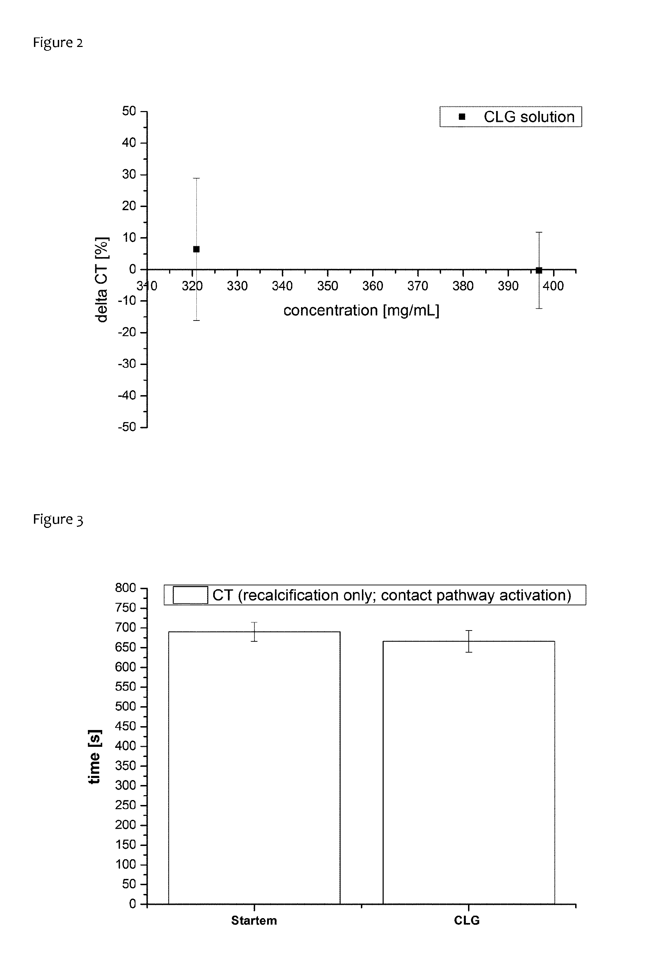

In detail, 300 μL citrated whole blood were taken, mixed with 4 μL of the respective solution of CLG of each concentration or of the star-tem@ reagent, and 16 μL of a 0.9% (m/V) NaCl-solution in ultrapure water. Blood clotting was initiated using the tissue factor based extem® reagent. The CLG solution has no negative influence on the initiation of coagulation reflected in the clotting time (CT), and there was no significant (student t test; probability p = 0.05, n = 10) difference between the CLG solution (396 mg/mL) and the star-tem@ solution. Therefore it is possible to recalcify standard citrated whole blood and restore its clotting ability by using CLG solutions. This is depicted in Figure 2, wherein the data of a ROTEM experiment using two differently concentrated solutions of CLG (320 mg/mL and 396 mg/mL) were used. The results in Figure 2 are given as a difference (in %) to the standard ex-tem® assay using star-tem@ recalcification (20μL startem® solution + 20μL extem® solution, i.e. solution containing tissue factor, in order to initiate coagulation; CT = 38 - 79s (according to specification)), and the bars represent the standard deviation (n = 10).

In addition the clotting properties were investigated when coagulation of whole blood (using 300μL of citrated whole blood) was not initiated using tissue factor but only contact pathway activation, i.e. the citrated whole blood was recalcified using either the star-tem@ reagent or a CLG solution. The measurement was performed according to the automated ROTEM® sequence. Normal values were taken from the "expected values" section of the "instructions for use" sheet provided together with the star-tem@ reagent. A comparison was carried out with the star-tem@ reagent and the CLG solution with a concentration of 396 mg/mL of example 2. As can be seen from Figure 3, the clotting time for both recalcification reagents is about 700 s, which is a normal value when coagulation is not accelerated but only initiated by contact to charged surfaces, i.e. the wall of the ROTEM® cup / pin. The difference between clotting time is around 3%, and CLG does not significantly (student's t-test; p = 0,05, n =10) differ from the star-tem@ reagent. All other parameters observed (i.e.: clotting time (CT), clot formation time (CFT), alpha angle (alpha), amplitude after 10 minutes (A10), maximum clot firmness (MCF),) with the ROTEM® device were also in a normal range (t (ROTEM® analysis) > 60 minutes) and did not differ from star-tem®, as can be seen from the following table 1.

Table 1: Measurement values of the data determined in Example 2

| Value | CLG | standard deviation (sdv) | Startem® | sdv | Normal range |
|---|---|---|---|---|---|
| CT | 666.00 | 27.22 | 690.25 | 24.31 | 300-1000 |
| CFT | 183.00 | 8.72 | 177.25 | 1.89 | 150-700 |
| alpha | 54.67 | 1.53 | 56.5 | 0.71 | 30-70° |
| A10 | 42.33 | 1.53 | 43.5 | 1.29 | 35-60 |
| MCF | 51.33 | 1.53 | 52.25 | 1.26 | 40-65 |

Example 3: State of the reagent

Another very important issue is the way of producing the solid reagent. In standard activated coagulation assays the blood is clotting within approximately 1 minute. That means blood needs a very fast recalcification in order to restore the factor's binding ability to membranes and to start the coagulation process. Therefore freeze drying of the compound was chosen for production to achieve dissolution within a few seconds and immediate disintegration. The dissolution rate can be described by Noyes Whitney equation:

$$-\frac{dm}{dt} = \frac{A \cdot D(c_s - c_t)}{\delta}$$

In this equation $-dm/dt$ is the decrease in mass over time due to dissolution, i.e. the dissolution rate, $A$ is the specific surface area, $D$ is the coefficient of diffusion through a diffusion layer (between non-dissolved solid and the solution/solvent) with thickness $\delta$, $c_s$ is the concentration in a saturated solution and $c_t$ is the concentration at time t. Based on the fact that the system is not stirred, $\delta$ becomes constant, and so are $D$ and $c_s$. Freeze dried products have a very high specific surface area when dried appropriately, i.e. no collapse occurs during the process. Therefore $A$ becomes very high and the dissolution rate will increase compared to a dry solid.

When $CaCl_2$ is freeze dried using very high concentrations it will decrease the melting point and/or the glass transition of the frozen system massively. This leads to very slow drying due to low temperatures and favors meltback, i.e. melting of the $CaCl_2$ if drying is not carried out adequately, or collapse during the freeze-drying process, leading to mechanical instability and bad dissolution. In addition $CaCl_2$ tends to crystallize incompletely and disturbs crystallization of other excipients, e.g. bulking agents, as described in Chang, B.S. and C.S. Randall, Use of subambient thermal analysis to optimize protein lyophilization; Cryobiology, 1992;. 29(5): p. 632-656. Aside from complicating the process, the hygroscopicity becomes even more relevant when $CaCl_2$ shall be freeze-dried because the high surface area will enhance water absorption, leading to much faster deliquescence.

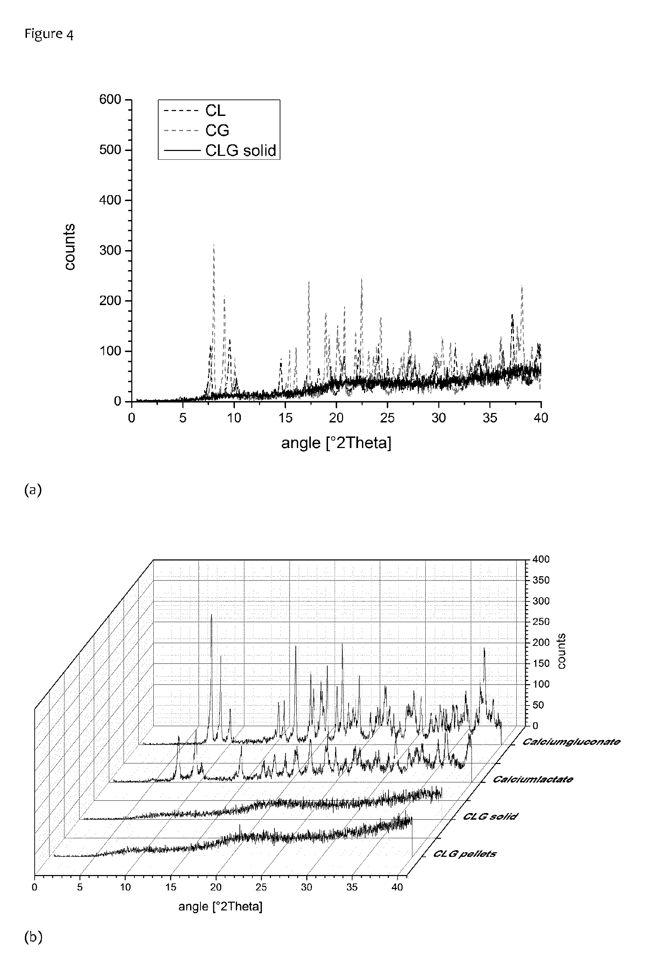

Since the CLG taken from Example 1 is produced by evaporation from an aqueous solution using a rotary evaporator, the resulting solid is completely amorphous, as can be seen from X-ray powder diffraction (Philips X'pert MPD X-Ray powder diffractometer with Cu K$\alpha$ radiation at 40 kV/40 mA and 25 °C; all samples were measured in a range $2\theta = 0.5°$ - 40°) diagrams of the produced CLG compared to the two educts calcium lactate x 1.5 $H_2O$ and calcium gluconate x 1 $H_2O$ used in Example1, the results of which are depicted in Figure 4a. As can be seen from the diagrams, CLG was present as raw product in amorphous form (CLG solid) as no clear peaks - which result from a crystal structure as in the educts calcium lactate and calcium gluconate - were observed.

Further, a part of the sample of CLG with a concentration of 320 mg/mL from Example 1 was freeze-dried under the following conditions:

Droplets are frozen in a stainless steel container filled with liquid nitrogen (T ~ -196°C).

The container is transferred to a precooled freeze dryer ($T_{inlet}$ (inlet temperature of the shelves)=-30°C). A Virtis™ Genesis (SP scientific) freeze dryer was used to remove the solvent (i.e. water). Primary drying is conducted at $T_{inelt}$ = -30°C and a chamber pressure of 30 mtorr for 15 hours. After finishing primary drying the shelves are heated (0.3°C/min.) until secondary drying inlet temperature $T_{inlet}$ = 40°C is reached. Secondary drying is also conducted at a chamber pressure of 30 mtorr for 4 hours.

As can be seen from Figure 4b, CLG was maintained in amorphous form even after freeze-drying (CLG pellets), showing no clear peaks in the diagram. The amorphous freeze dried product cannot be produced by skipping the rotary evaporation and freeze dry a solution containing both excipients, calcium lactate x 1.5 $H_2O$ and calcium gluconate x 1 $H_2O$, due to their low solubility.

The reason for the formation of an amorphous product seems to be the highly oversaturated solution formed during the rapid evaporation / dehydration due to the high temperature and the constant movement of the flask combined with low pressure. This solution does not crystallize but solidifies forming an amorphous solid [Inagaki, M. (1977). "Some Aspects on the Optical Resolution by Preferential Crystallization based on Phase Equilibrium". Chemical and Pharmaceutical Bulletin 25(10): 2497-2503]. Amorphous solids contain an excess in Gibbs energy compared to their crystalline counterparts and are therefore metastable. In this case the amorphous solid is kinetically stabilized due to rapid dehydration, but it still can release its stored energy through crystallization.

Nucleation and therefore crystallization occurs preferentially at temperatures between the glass transition temperature (Tg) (also called glass temperature in the following) and the melting point of the amorphous solid and the corresponding crystalline solid respectively. The glass transition temperature of a mixture of two or more amorphous components within one compound can be calculated by the "Fox - equation" a deviation from the "Flory - Fox - equation" [Fox , T. G. (1956). "Influence of diluent and of copolymer composition on the glass temperature of a polymer system". Bull. Am. Phys. Soc. (1) page 123].

$$\frac{1}{T_g} = \sum_{i=1}^{n} \frac{\omega_i}{T_{g_i}}$$

Where Tg is the glass transition temperature of the system; $\omega_i$ are the weight percent of the component i and $Tg_i$ is the glass transition of component i. This approach also fits the often used Gordon -Taylor equation (Gordon, M. and J. S. Taylor (1952). "Ideal copolymers and the second-order transitions of synthetic rubbers. i. non-crystalline copolymers." Journal of Applied Chemistry 2(9): 493-500) applicable for a two component system, assuming k = Tg1/Tg2.

$$T_g = \frac{\omega_1 T_{g,1} + k\omega_2 T_{g,2}}{\omega_1 + k\omega_2}$$

Concerning this relationship and the fact that crystallization rate increases over Tg it is obvious that a high Tg is of crucial importance for a stable product and especially for long term stability. In addition to recrystallization, molecular mobility is important for long term stability of an amorphous solid. The temperature where molecular motion becomes negligible is called "Kauzmann temperature". This temperature threshold can be determined using thermal analysis or can be roughly calculated. The following well established empirical rule [Hancock, B. C. and G. Zografi (1997). "Characteristics and significance of the amorphous state in pharmaceutical systems". Journal of pharmaceutical sciences 86(1): 1-12] provides an orientation for the "Kauzmann temperature" and therefore for the storage temperature:

$$T_{storage} \leq T_g - 50K$$

Not only the composition but also residual moisture content influences Tg of the final system. Due to its very low Tg (136K) [MacFarlane, D. R. and C. A. Angell (1984). "Glass transition for amorphous solid water". The Journal of Physical Chemistry 88(4): 759-762] water is a powerful plasticizer which can decrease Tg of the final product dramatically, depending on its weight percent, i.e. residual moisture content.

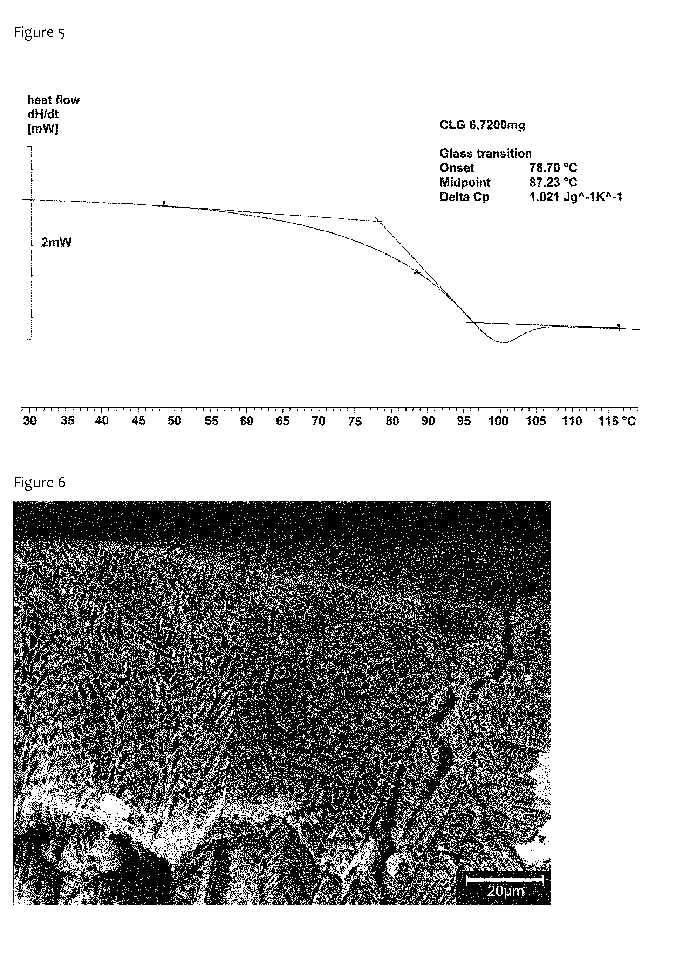

The following data using differential scanning calorimetry analysis (DSC 822e, Mettler Toledo connected to a computer with STARe software (for data analysis); Heating rate / cooling rate = 10°C/min. with an isothermal equilibration step between heating and cooling of 2 min.) of CLG, shown in Figure 5 for CLG with a molar ratio of 1:1 (of Example 1), support the theory that an amorphous solid is formed. To determine residual moisture contents of the substances, a Karl - Fischer device with electronic dead stop method (831 KF Coulometer and 832 KF Thermoprep (oven) produced by Methrom (also termed KF-apparatus)) was used. Hydranal® Coulomat AG Oven served as a reagent. About 10 mg of the substance was used for an expected residual moisture between 5 - 10%(m/m) and about 50 - 80 mg were used for an expected moisture content $\geq$ 1% (m/m).

In addition to the above mixture, amorphous solids of CLG with different molar ratios and residual moisture content have been produced for comparing the results in differential scanning calorimetry to see the influence of these parameters. As a result the following was observed:

The final solid product obtained after the above freeze-drying procedure shows a clear glass transition which depends on residual moisture content and the composition of CLG, i.e. the weight ratio between calcium lactate and calcium gluconate. The glass transition was found at about 84.90 °C and 87.20 °C, for a 2:1 (CL:CG) and a 1:1 (CL:CG) mixture, respectively, at similar moisture contents (6.52 % [m/m] and 5.71 % [m/m] respectively). The influence of the moisture content can be seen in the following table 2.

Table 2: Influence of moisture content on Tg for different ratios of CL and CG in CLG

| CLG ratio [%(m(CL)/m(CLG)] = 100 | | 62.23 | | 50.44 | | 28.93 | | 0 | |
|---|---|---|---|---|---|---|---|---|---|
| Moisture content [%(m/m)] | Tg [°C] | Moisture content [%(m/m)] | Tg [°C] | Moisture content [%(m/m)] | Tg [°C] | Moisture content [%(m/m)] | Tg [°C] | Moisture content [%(m/m)] | Tg [°C] |
| 0* | 152.06 | 0 | 144.37 | 6.52 | 84.9 | 0 | 125.01 | 0 | 110.03 |
| 5.20 | 112.86 | 10.00 | 58.91 | - | - | 4.54 | 92.14 | 3.69 | 79.16 |
| 10.15 | 58.45 | 10.37 | 56.54 | - | - | 5.71 | 87.20 | | |
| 14.00 | 34.39 | - | - | - | - | 8.30 | 61.35 | 9.86 | 29.77 |
| - | - | - | - | - | - | 9.91 | 40.75 | 14.30 | 14.26 |
| *: this initial value was produced by sample annealing below Tg within the KF-apparatus until baseline drift was reached again, i.e. no residual water detectable. | | | | | | | | | |

As already observed above, the data obtained with X - ray powder diffraction confirm the amorphous state of the solid (see Figure 4). There is a clear amorphous fraction and no crystalline structure detectable for both, the initial solid produced by evaporation and the final freeze dried product. Therefore the obtained metastable system does neither stabilize itself during the freezing step by crystallization of the different excipients nor does it change its properties during the drying step, i.e. no crystallization occurred due to annealing effects, so-called sub Tg' annealing. Amorphous pellets were obtained after freeze drying.

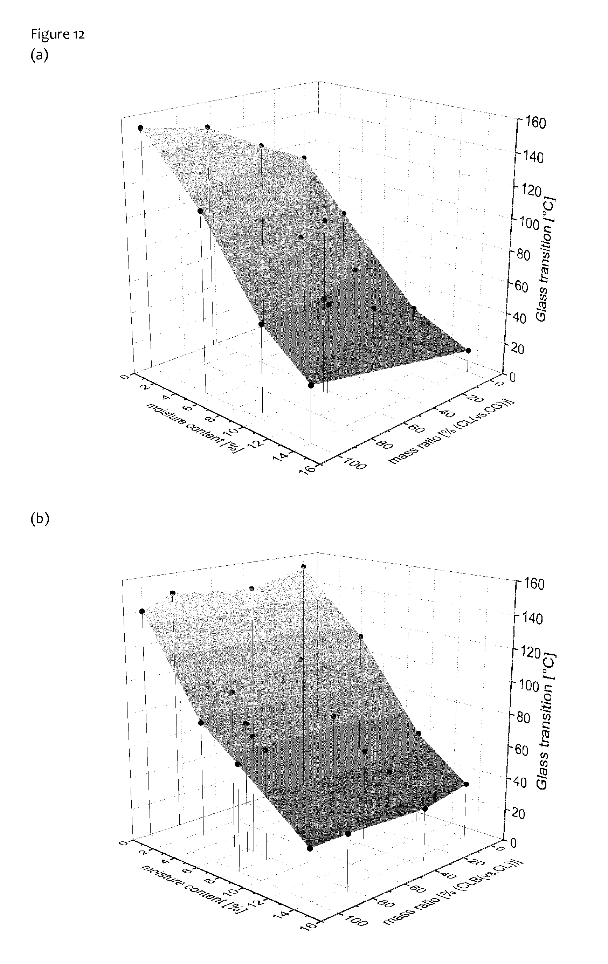

Figure 12a also shows the influence of different compositions and residual moisture content on the glass transition temperature of the CLG compound.

In contrast to $CaCl_2$ CLG remains completely amorphous during freeze-drying, as was observed when comparing the above data of CLG to freeze-dried $CaCl_2$.

The maximally frozen system of CLG with a molar ratio of 1:1 formed from a solution containing 320 mg/mL CLG has a glass transition temperature Tg' of - 11.74 °C, so primary drying can be carried out at relatively high temperatures (upon optimization of the drying cycle even higher than - 10°C due to sublimation and thus sinking temperature of the pellet), leading to faster drying and shorter cycles.

An optical analysis of the freeze-dried product (c = 320 mg/mL) using scanning electron microscopy (AMray 1810 T scanning electron microscope) revealed the highly porous structure, as shown in Figure 6. In addition, no collapse or meltback could be detected when CLG was freeze-dried appropriately. CLG is, however, evidently metastable as a solid, i.e. amorphous. The dissolution rate of the freeze-dried product was very high so the solid disintegrated immediately when it came in contact with a water surface and dissolved rapidly and completely after a few seconds.

Example 4: Coagulation experiment with CLG

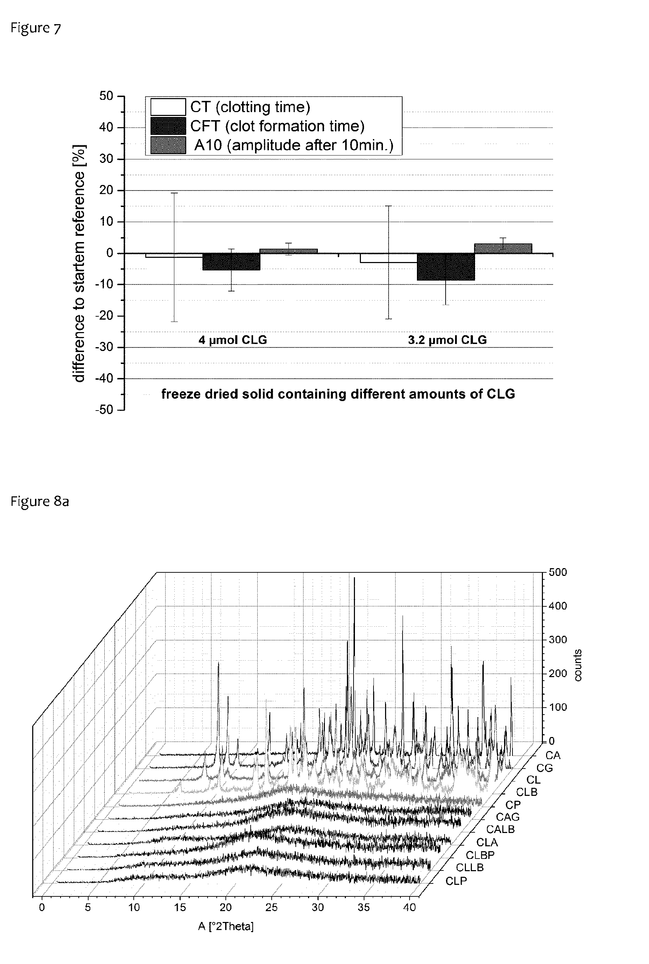

The compatibility with coagulation measurement was determined using the ROTEM® device. The measurement was conducted according to the respective automated ROTEM® sequence. 300 $\mu$L citrated whole blood (obtained from healthy individuals) were used for each measurement. The pipetting of the respective liquid reagents was performed according to the sequence or was skipped for the solid reagents. Figure 7 shows the results of an analysis where freeze-dried solids (pellets) containing different amounts of CLG, produced by the method described in Example 3, were added to a cup immediately before measurement instead of a $CaCl_2$ solution in order to recalcify a citrated whole blood sample, wherein 9 mL whole blood were collected in Sarstedt coagulation 9 NC tubes containing 1 mL 0,016 M sodium citrate solution prior to the addition. Coagulation was initiated using the tissue factor based ex-tem® reagent. As a reference the standard ex-tem® assay was performed using 20$\mu$L of the star-tem® reagent (200mM $CaCl_2$ solution) to recalcify the WB. The 4$\mu$mol equivalent correlates with the amount of $Ca^{2+}$ added through star-tem® in a standard assay. In addition, experiments were conducted with freeze-dried $CaCl_2$ pellets, produced by the same method as described above, which, however, liquefied rapidly due to the high hygroscopicity of the pellets.

The ex-tem® assay was selected because of the tissue factor coagulation activation and the resulting short clotting times (CT). Therefore it was possible to investigate whether the freeze-dried products containing a high amount of solid would dissolve rapidly enough to assure complete recalcification and clotting times within a normal range. The results in Figure 7, which are depicted in comparison to the reference (with deviation from the reference shown in %), show that there was no significant (p = 0.05; n = 10) difference between the CT values of the samples recalcified with CLG and the reference samples. Neither the clot formation times (CFT) nor the amplitudes after 10 minutes reaction time (A10) of the 4$\mu$mol equivalent did significantly (p = 0.05, n = 10) differ from the reference. Although there is not a specific concentration but a range regarding recalcification, the lower amount of CLG added to recalcify the sample showed slight but significant (p = 0.05, n = 10) variations in CFT and $A_{10}$ when compared to the reference. Thus it was shown that an amount of reagent comparable to star-tem® (corresponding to about 4 $\mu$mol $Ca^{2+}$) is preferably added, although the system tolerated some deviation. The higher solid content had, however, no negative influence regarding disintegration, and therefore it is possible to add the required amount of $Ca^{2+}$ by adding the respective mass of freeze-dried CLG to the sample.

The data show that CLG evidently outclasses $CaCl_2$ regarding production and handling and provides a clear advantage in in-vitro coagulation measurements due to the possibility to produce solid reagents for recalcification.

Example 5: Production of further reagents

Calcium lacto ascorbate (CLA), calcium lacto lactobionate (CLLB), calcium lacto pantothenate (CLP), calcium ascorbate lactobionate (CALB), calcium lactobionate pantothenate (CLBP),and calcium ascorbate gluconate (CAG) have been produced from equimolar amounts (molar ratio 1 : 1) of calcium lactate (CL), calcium ascorbate (CA), calcium lactobionate (CLB), calcium gluconate (CG) and calcium pantothenate (CP), respectively, by the same method as described in Example 1. In addition, a further calcium lacto gluconate with a molar ratio of CL : CG of 0.8 to 1 was produced, which was termed CLG ME. Additionally, a sample was produced using calcium lactate, calcium gluconate and calcium chloride (CLGCl2) at a molar ratio CL: CG : CaCl$_2$ of 2:2:1.

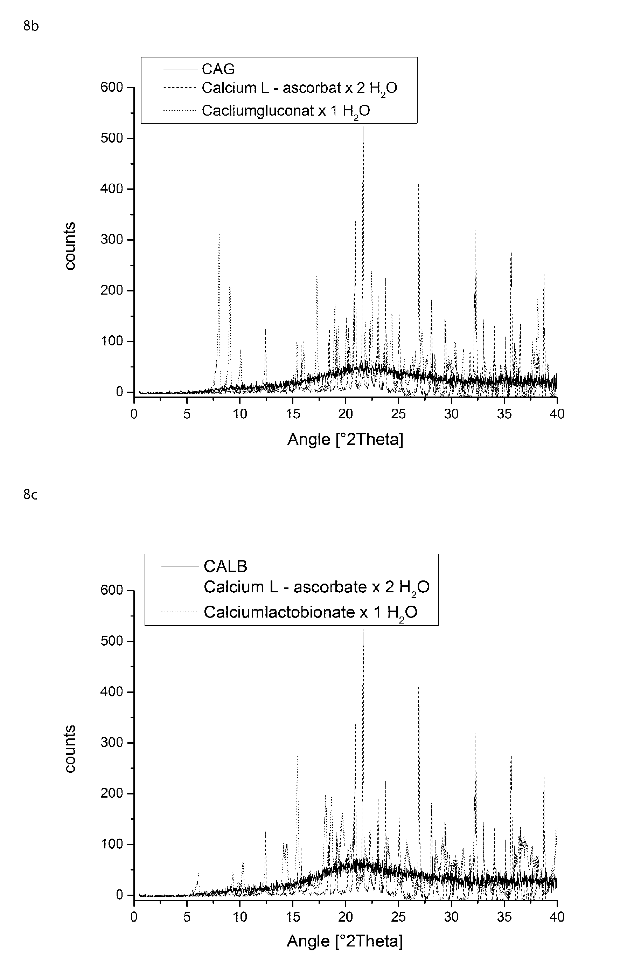

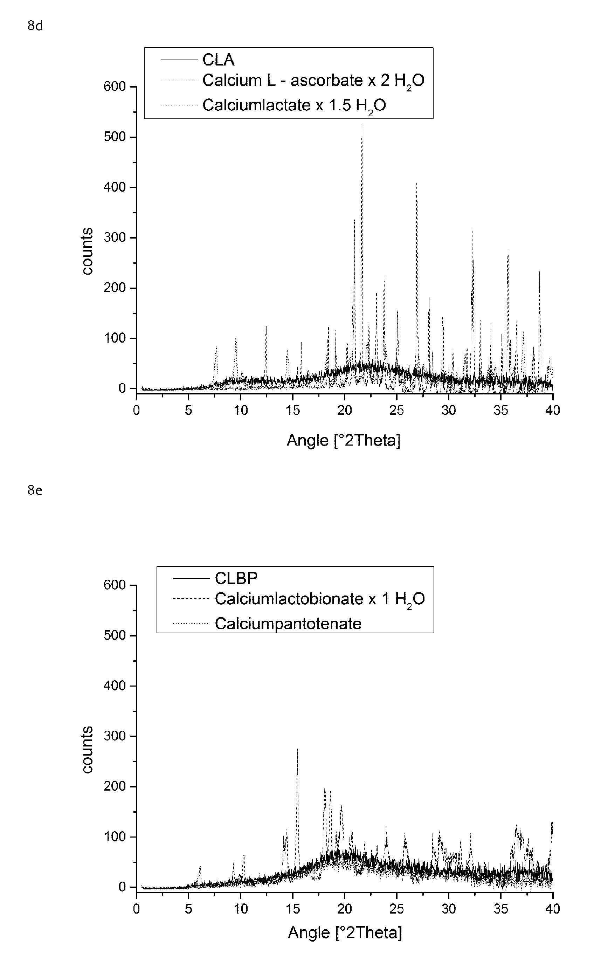

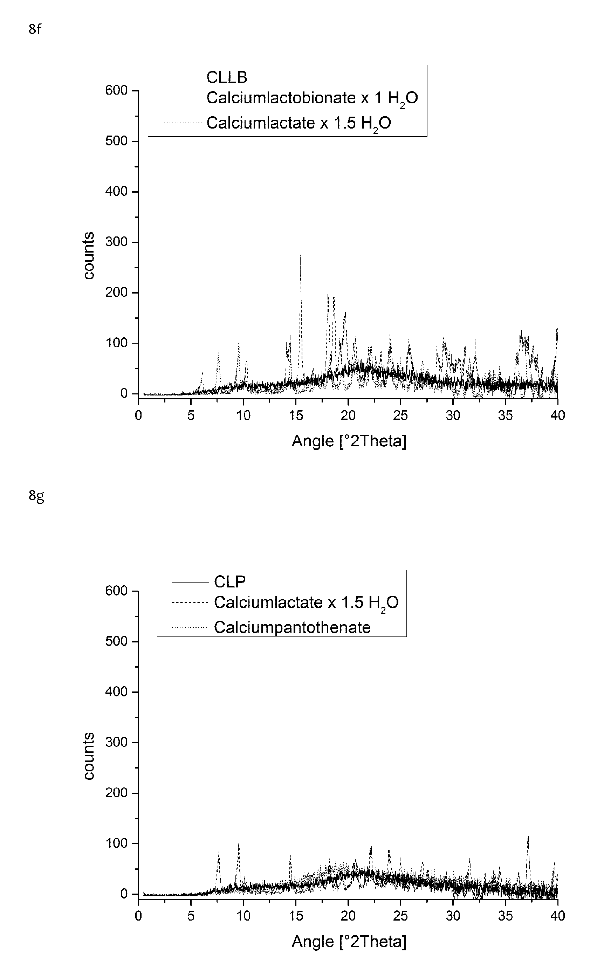

X-ray powder diffraction experiments were conducted with the resulting products and the educts in the same way as in Example 3. As can be seen from Figure 8, the resulting compounds CLA, CLLB, CLP, CALB, CLBP and CAG were obtained in amorphous form, while the educts CA, CG, CL and CLB were showing a crystalline structure. CP also was present in amorphous form.

Further, also the glass transition temperature was obtained for the resulting compounds in the same way as in Example 3, as well as the residual moisture. In addition, moisture uptake was determined using a modified method described in the European Pharmacopoeia Section 5.11. hygroscopicity. It determines the mass increase (% (m/m$_0$)) during a 24h storage period over a saturated ammonium sulfate solution. Approximately 100 mg of each substance were placed in a desiccator at 25°C in a climate chamber. The results thereof are shown in table 4.

In addition, in table 4 the value Mr refers to the calculated average molecular weight of the highly water soluble and low hygroscopic compound resulting from a mixture of two or more organic Ca$^{2+}$ salts that interact on the molecular level, which is calculated as follows:

$$Mr(compound) = \frac{\sum_i \chi_i M_i}{\sum_i \chi_i}$$

wherein M$_i$ is the respective molecular weight of the individual salt in the compound and $\chi_I$ is the respective molecular proportion (in mole) of the individual salt in the compound.

For example, in the case of CLG ME 4 parts of CL are added to 5 parts of CG, so that in average in 9 molecules of CLG ME 4 molecules are derived from CL and 5 molecules are derived of CG, wherein of course also mixed molecules with one calcium ion, one lactate ion and one gluconate ion can be present.

For this example, the following molecular mass would be obtained:

M (CL) anhydr. = 218,22 g/mol
M (CG) anhydr. = 430,39 g/mol
M (L$^-$) = 89,07 g/mol
M (G$^-$) =195,16g/mol
M (Ca$^{2+}$) = 40,08 g/mol

$$Mr\,(CLG\ ME) = \left(4 \cdot (2 \cdot M(L^-) + M(Ca^{2+})) + 5 \cdot (2 \cdot M(G^-) + M(Ca^{2+}))\right)/\,9 = 336.09\ g/mol$$

Further, the procedure described in the European pharmacopoeia section 5.11. solubility was performed for the amorphous components. 100mg finely powered substance (180$\mu$m < x < 90$\mu$m (determined by sieving: after milling with a pestle and a mortar the substance was sieved through a 180$\mu$m sieve (nylon; d = 20cm), the fraction which passed the 180$\mu$m sieve was placed on a 90$\mu$m sieve (stainless steel, d = 20cm). The substance used for analysis was the fraction which remained on the second sieve (90$\mu$m) after passing the first (180$\mu$m)) of the respective substance. TRIS buffer (isotonic, pH = 7.4 at 25°C) was used as dissolution medium. The solubility is determined visually after 1 min. mixing and 15 min. storage at the respective temperature. The experiments were carried out using a water bath set to 25.0°C. The criteria for determining the solubility category are given in table 3.

Table 3: Categorization of solubility according to Ph. Eur. 7.4

| Substance [mg] | Added volume [mL] | Solubility (s) [g/L] | Category |
|---|---|---|---|
| 100* | 0.1 | $\geq 1000$ | Very soluble |
| 100* | 1.0 | $1000 > s \geq 100$ | Freely soluble |
| 100* | 3.0 | $100 > s \geq 33.3$ | Soluble |
| 100* | 10.0 | $33.3 > s \geq 10$ | Sparingly soluble |
| 10 | 10.0 | $10 > s \geq 1$ | Slightly soluble |
| 1 | 10.0 | $1 > s \geq 0.1$ | Very slightly soluble |
| If 1mg of the substance does not dissolve in 10.0 mL | | | Practically insoluble |

The data for the measurements in Example 5 are shown in the following table 4. In regard to table 4, it should be noted that calcium chloride did not reach equilibrium for the moisture uptake measurement.

The calculated solubility range ($C_{max} > C_s \geq C_{min}$) in table 4 was calculated according to the following equations:

$$C_{max} \text{ (mol/L)} = C_{max} \text{ (g/L taken from table 3 for the respective solubility)} / Mr$$

$$C_{min} \text{ (mol/L)} = C_{min} \text{ (g/L taken from table 3 for the respective solubility)} / Mr$$

Table 4: Summary of data of Example 5

| Substance | Mr [g/mol] (referring to 1 mole $Ca^{2+}$) | Residual moisture [% $\pm$ sdv] (m/m) | Structure after production | Tg (at the given moisture content) [°C] | Ph. Eur. method | | Calculated solubility $C_s$ [mol/L] |
|---|---|---|---|---|---|---|---|
| | | | | | Hygroscopicity [%(m/m$_o$] $CaCl_2$ = 113.9% | Solubility (see above) $CaCl_2$ = freely soluble | |
| CA | 390.34 | 4.68$\pm$0.81 | amorphous | 72.3 | 31.77 | very soluble | $C_s \geq 2,562$ |
| CG | 430.39 | 3.69$\pm$0.16 | amorphous | 79.2 | 20.16 | sparingly soluble*** | $0.077 > C_s \geq 0.023$ |
| CL | 218.22 | 5.18$\pm$0.90 | partially crystalline** | 112.9 | 30.28 | soluble | $0.468 > C_s \geq 0.156$ |
| CLB | 754.68 | 5.10$\pm$0.70 | amorphous | 82.5 | 21.08 | very soluble | $C_s \geq 1.325$ |
| CP* | 476.54 | | amorphous | | 22.02 | freely soluble | $2.100 > C_s \geq 0.210$ |
| CLG ME | 324.96 | 5.71 $\pm$ 0.51 | amorphous | 87.2 | 19.91 | very soluble | $C_s \geq 3.077$ |
| CLG (2:1) | 288.94 | 6.52 $\pm$ 0.08 | amorphous | 84.9 | 20.44 | freely soluble | $3.460 > C_s \geq 0.346$ |
| $CLGCl_2$ | 281.6 | 10.32 $\pm$ 0.29 | amorphous | 62.6 | 20.24 | very soluble | $C_s \geq 3.551$ |
| CLA | 304.71 | 5.04 $\pm$ 0.15 | amorphous | 66.2 | 30.51 | very soluble | $C_s \geq 3.288$ |
| CLLB | 486,45 | 6.67 $\pm$ 0.87 | amorphous | 57.4 | 19.18 | freely soluble | $2.060 > C_s \geq 0.206$ |
| CLP | 347,38 | 4.36 $\pm$ 0.09 | amorphous | 67.6 | 27.20 | freely soluble | $2.880 C_s \geq 0.288$ |
| CAG | 410.37 | 5.43 $\pm$ 0.58 | amorphous | 67.2 | 19.65 | freely soluble | $C_s \geq 2.437$ |
| CALB | 572.51 | 5.04 $\pm$ 0.38 | amorphous | 76.8 | 23.63 | very soluble | $C_s \geq 1.747$ |

(continued)

| Substance | Mr [g/mol] (referring to 1 mole $Ca^{2+}$) | Residual moisture [% $\pm$ sdv] (m/m) | Structure after production | Tg (at the given moisture content) [°C] | Ph. Eur. method | | Calculated solubility $C_s$ [mol/L] |
|---|---|---|---|---|---|---|---|
| | | | | | Hygroscopicity [%(m/m$_o$] $CaCl_2$ = 113.9% | Solubility (see above) $CaCl_2$ = freely soluble | |
| CLBP | 615.61 | 3.71 $\pm$ 0.41 | amorphous | 85.6 | 25.39 | very soluble | $C_s \geq 1.624$ |

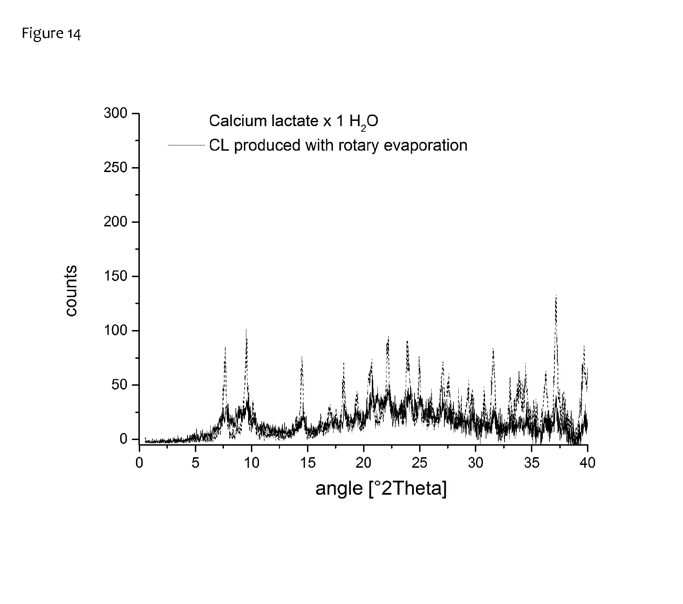

*: Raw material received from manufacturer is amorphous
**: XRD results show crystallization of CL (see figure 14), characteristic peaks at 7.7°2θ, 9.6°2θ and 14.6 °2θ; in addition a Tg at 112.9°C was detectable using DSC.
***: Crystallization between 10 minutes and 15 minutes. Completely dissolved after 10 min. The solubility of the crystallizing substance is identical with the value determined for calciumgluconate x 1 $H_2O$(cryst.) using the same method.

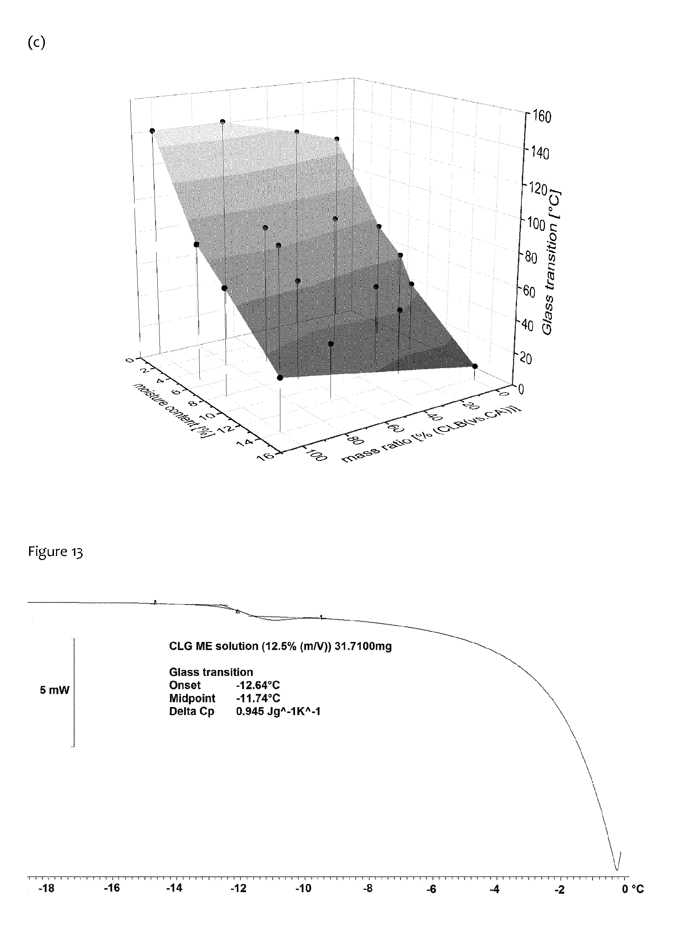

All of the substances produced with the rotary evaporation method were also freeze dried except for CL, due to rapid crystallization. The substances were dissolved in filtrated (0.23$\mu$m) bi-distilled water using 12.5 % (m/V) solid content. The resulting solutions were analyzed using the DSC in order to determine the glass transition of the maximally frozen system (Tg'), which is an important parameter for the freeze drying process (Franks, F. (1997). "Freeze-drying of bioproducts: putting principles into practice." European Journal of Pharmaceutics and Biopharmaceutics 45((1998)): 221 - 229.). An example for a DSC thermogram showing the Tg' of a CLG ME solution (12.5% (m/V)) is given in figure 13.
Primary drying was carried out at $T_{inlet}$ = -30° C / 30mtorr for 15h followed by secondary drying at 40°C / 30mtorr for 6h with a heating rate between the two steps of 0.3 °C/min. The glass transition temperature as well as the residual moisture content were measured after freeze drying for the respective substance. The results are shown in Table 5.

Table 5: Tg' values for 12.5% (m/V) solutions of the respective substance and Tg values for the resulting freeze dried product.

| Substance | Glass transition of the frozen system* (Tg') [°C] | Glass transition after freeze drying (Tg) [°C] | Residual moisture content [%(m/V)] | sdv. |
|---|---|---|---|---|
| CA | -24.61 | 123.02 | 0,709 | 0,021 |
| CG | -11.08 | 103.05 | 0,727 | 0,014 |
| CL | n.d.** | n.d.** | n.d.** | n.d.**- |
| CLB | -13.27 | 136.51 | 0,706 | 0,014 |
| CP* | -25.28 | 74.77 | 0,767 | 0,002 |
| CAG | -15.70 | 101.81 | 0,744 | 0,009 |
| CALB | -13.28 | 126.53 | 0,738 | 0,017 |
| CLA | -20.18 | 130.68 | 0,735 | 0,030 |
| CLBP | -18.84 | 114.21 | 0,746 | 0,074 |
| CLG ME | -11.74 | 118.53 | 0,741 | 0,003 |
| CLG J | -13.50 | 120.50 | 0,745 | 0,040 |
| CLLB | -15.58 | 132.21 | 0,722 | 0,006 |
| CLP | -23.02 | 103.28 | 0,742 | 0,021 |

*: solutions contained 12.5% (m/V) of the respective amorphous solid
**: Tg' determination and manufacturing not possible due to rapid crystallization

CLLB and CALB were selected for further investigation to guarantee that the "Fox - equation" is also valid for other systems than CLG. As for CLG, the influence of different compositions and residual moisture content was investigated. The results are summarized in figure 12b and 12c for CLLB and CALB, respectively, as well as tables 6 and 7.

Table 6: Influence of moisture content on Tg for different ratios of CL and CLB in CLLB

| CLLB ratio [%(m(CL)/m(CLB)] = 100 | | 82.77 | | 34.82 | | 0 | |
|---|---|---|---|---|---|---|---|
| Moisture content [%(m/m)] | Tg[°C] | Moisture content [%(m/m)] | Tg[°C] | Moisture content [%(m/m)] | Tg[°C] | Moisture content [%(m/m)] | Tg[°C] |
| 0* | 140.08 | 0 | 148.05 | 0 | 143.05 | 0 | 152,055 |
| 5.10 | 82.46 | 5.19 | 96.38 | 4.44 | 104.03 | 5,196 | 112,86 |
| 8.20 | 64.81 | 6.35 | 79.84 | 7.26 | 73.08 | 10,151 | 58,45 |
| 13.87 | 30.65 | 6.93 | 73.34 | 9.86 | 56.42 | 14 | 34,39 |
| - | - | 8.00 | 67.39 | 11.82 | 47.945 | - | - |
| - | - | 14.49 | 34.39 | 14.60 | 32.13 | - | - |
| *: this initial value was produced by sample annealing below Tg within the KF-apparatus until baseline drift was reached again, i.e. no residual water detectable. | | | | | | | |

Table 7: Influence of moisture content on Tg for different ratios of CA and CLB in CALB

| CALB ratio [%(m(CA)/m(CLB)] = 100 | | 64.23 | | 23.54 | | 0 | |
|---|---|---|---|---|---|---|---|
| Moisture content [%(m/m)] | Tg[°C] | Moisture content [%(m/m)] | Tg[°C] | Moisture content [%(m/m)] | Tg[°C] | Moisture content [%(m/m)] | Tg[°C] |
| 0* | 140.09 | 0 | 140.90 | 0 | 128.97 | 0 | 121.13 |
| 5.10 | 82.46 | 4.82 | 84.55 | 4.30 | 81.00 | 4.68 | 72.34 |
| 8.20 | 64.81 | 6.29 | 77.15 | 8.62 | 48.33 | 6.93 | 58.83 |
| 13.87 | 30.65 | 8.32 | 60.75 | 10.95 | 40.12 | 8.09 | 42.97 |
| - | - | 11.68 | 32.61 | - | - | 14.18 | 8.59 |
| *: this initial value was produced by sample annealing below Tg within the KF- pparatus until baseline drift was reached again, i.e. no residual water detectable | | | | | | | |

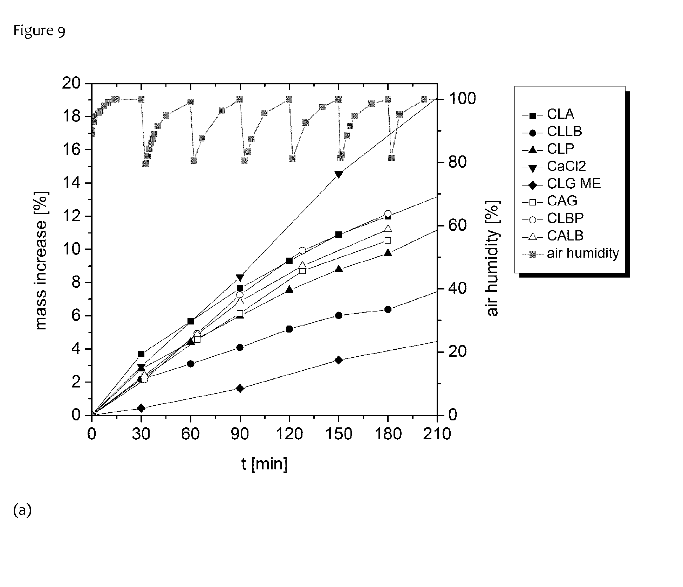

In addition, hygroscopicity measurements have also been carried out with the substances by a method similar to the one in Example 1. Samples of CLA, CLLB ,CLP, CAG, CALB and CLBP as well as $CaCl_2$ and CLG were placed in an exsiccator over a saturated $K_2SO_4$ solution at a relative humidity of about 100% and then into a conditioning cabinet at about 25°C. At such humidity even substances that have low hygroscopicity take up humidity and liquefy, like e.g. $CaCl_2$ which requires a humidity of about 25%. The results of the measurements are shown in Figures 9 (a) to 9 (c).
An important factor is resorption speed of humidity in the initial range (3 hours) compared to $CaCl_2$, which can be seen from table 8.

Table 8: Results of resorption measurement

| Compound (amorphous except for $CaCl_2$) | Uptake rate during 3h storage at 99.9 % rel. humidity (sat. $K_2SO_4$ sol.) [ratio compared to $CaCl_2$] |
|---|---|
| $CaCl_2$ | 1.000 |
| CLA | 0.638 |
| CLBP | 0.637 |

(continued)

| Compound (amorphous except for CaCl$_2$) | Uptake rate during 3h storage at 99.9 % rel. humidity (sat. K$_2$SO$_4$ sol.) [ratio compared to CaCl$_2$] |
|---|---|
| CALB | 0.629 |
| CAG | 0.612 |
| CLP | 0.536 |
| CA | 0.449 |
| CL | 0.384 |
| CLLB | 0.357 |
| CP | 0.299 |
| CLB | 0.226 |
| CG | 0.214 |
| CLG J | 0.213 |
| CLG ME | 0.204 |

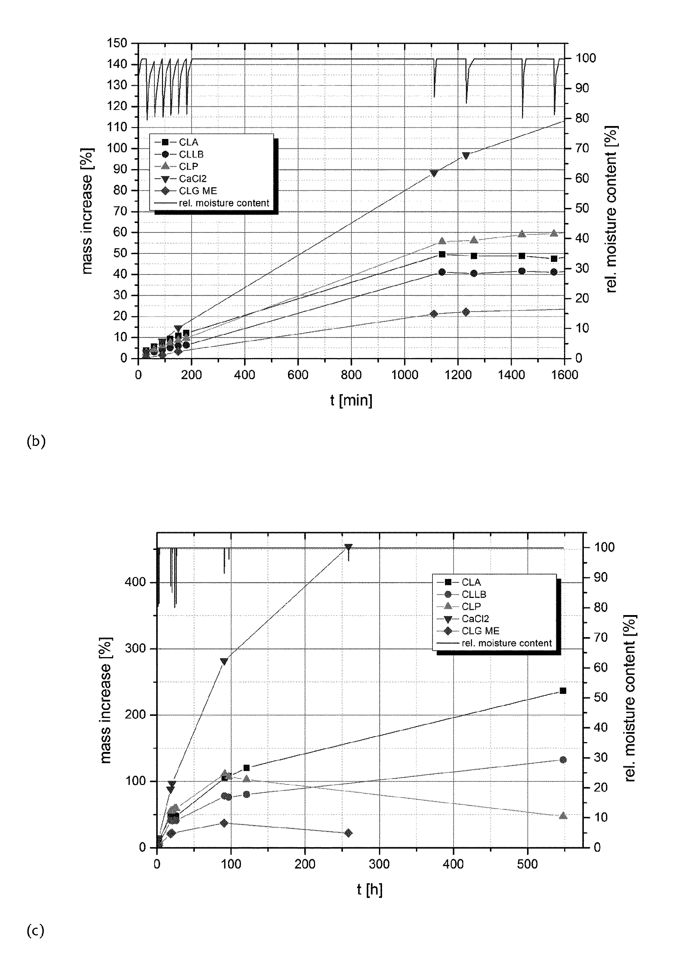

As can be seen from Figure 9 and tables 4 to 8 the compounds according to the invention also show improved resorption compared to CaCl$_2$. The best results were obtained with CLG, respectively CLG ME.

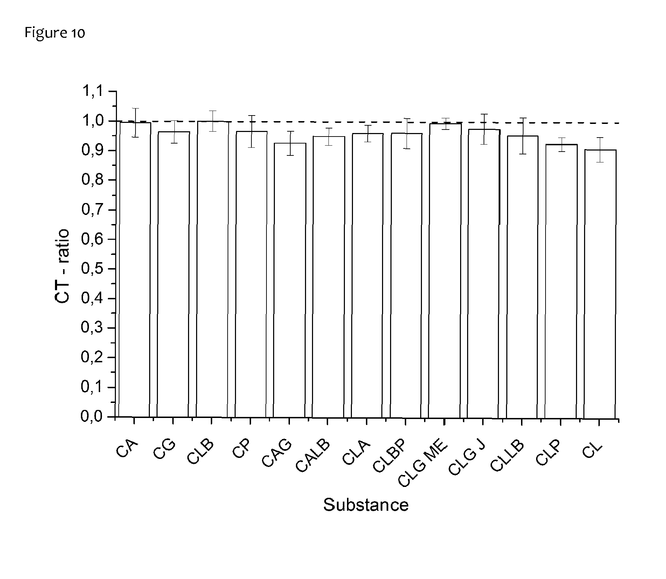

A coagulation essay (NATEM assay) was carried out with the produced samples in the same way as in Example 2. The results thereof are shown in Figure 10, wherein the results of each compound are shown as ratio compared to the standard NATEM® assay using star-tem®. As can be seen from the figure, also the further produced compounds do not negatively affect the measurement, wherein here exemplary the clotting time is shown, compared to the standard reagent.

Example 6: Use of different CLG

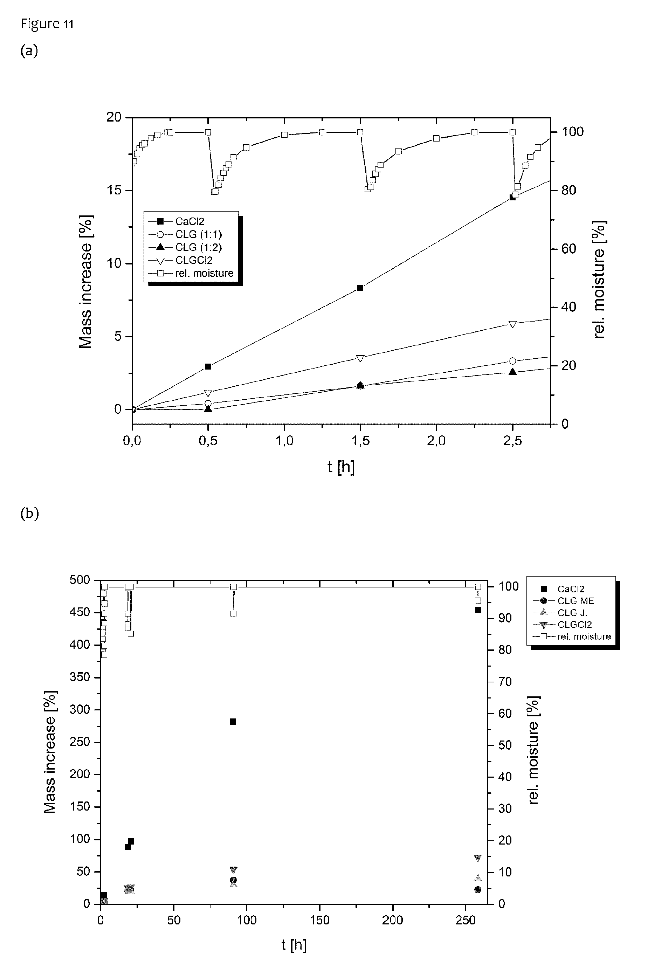

CLG solids with a ratio of 2:1 and 1:1 prepared as in Example 1, CaCl$_2$ as reference and CLG ME and CLGCl$_2$ from Example 5 were compared regarding their hygroscopicity using the method described in Example 1. In addition, a commercial CGL from Jungbunzlauer (CLG J) with an approximate ratio of 2:1 was used for comparison. The results are shown in Figure 11. As can be seen from the figure, all CLG samples showed improved hygroscopicity behavior compared to CaCl$_2$.

Example 7: Disintegration / dissolution time of different compounds

Disintegration of substances described in Examples 5 and 6 were determined according to the method described in the European Pharmacopoeia 7.4 in monograph "tablets" section "oral lyophilisates". The disintegration time is acceptable if the oral lyophilisate disintegrates within 3 minutes in 200mL of "purified water R" at temperatures between 15°C and 25°C. Herein, disintegration is the loss of the overall structure, whereas dissolution means that the compound is completely dissolved.

This method was adjusted for the small volume lyophilisates which were used here (~10 μL initial solution which corresponds to a lyophilysate mass of about 1 mg). Therefore the lyophilized product was added to 300 μL of TRIS buffer (adjusted pH 7.4 at 22°C) without any additional manipulation, e.g. shaking or stirring. In addition to the disintegration time the time until the whole product was completely dissolved, i.e. no visible particles detectable, has been determined at about 22°C (22.1 - 22.3 °C during the experiment).

The following table 3 sums up the results for disintegration time / dissolution time of the lyophilisate produced using the respective substance at a concentration of 12.5 % (m/V) solid content. All of the substances were produced by the method described above at a molar ratio of 1:1 (in this Example) for the different compounds, except CLG ME and CLG J. In addition the excipients (i.e. calcium ascorbate, calcium gluconate, calcium lactobionate and calcium pantothenate) were produced in the same way using also 12.5% (m/V) total solid content using the respective amorphous substance produced by the described above. It was not possible to lyophilize pure amorphous calcium lactate due to rapid solidification and partially crystallization of the solution, which contained also 12.5%(m/V) amorphous calcium lactate. Results are shown in table 9.

Table 9: Disintegration time and dissolution time for substances

| Lyophilized substance (mean mass: 1.14 ±0.04mg) | Disintegration time [s]* | Dissolution time [s] (mean ± sdv) |
|---|---|---|
| CA | <1 | 11 ± 1 |
| CG | <1 | 9 ± 1 |
| CLB | <1 | 13± 2 |
| CP | <1 | 6 ± 1 |
| CAG | <1 | 11 ± 2 |
| CALB | <1 | 12 ± 1 |
| CLA | <1 | 14 ± 3 |
| CLBP | <1 | 10 ± 2 |
| CLG ME | <1 | 19 ± 6 |
| CLG J | <1 | 25 ± 8 |
| CLLB | <1 | 18 ± 4 |
| CLP | <1 | 8 ± 1 |

*: all of the lyophilized products disintegrated immediately after addition to the dissolution medium

## Claims

1. A dry diagnostic reagent comprising at least a highly water soluble and low hygroscopic compound resulting from a mixture of two or more organic $Ca^{2+}$ salts that interact on the molecular level in a manner that at least partially prevents crystallization of the resulting compound.

2. The diagnostic reagent of claim 1, wherein the compound resulting from a mixture of two or more organic $Ca^{2+}$ salts that interact on the molecular level in a manner that at least partially prevents crystallization of the resulting compound has a solubility in water of at least 100 g/l, preferably at least 250 g/l, and more preferably of at least 400g/l, or wherein the compound resulting from a mixture of two or more organic $Ca^{2+}$ salts that interact on the molecular level in a manner that at least partially prevents crystallization of the resulting compound has a hygroscopicity as measured by mass increase at relative air humidity >50% of <5% mass over 96 hours, preferably <1% over 96 hours, and more preferably <0.1% over 96 hours.

3. The diagnostic reagent of one or more of the preceding claims, where the resulting compound is present in amorphous form.

4. The diagnostic composition according to one or more of the preceding claims, where at least one salt is formed by an organic acid.

5. The diagnostic composition according to claim 4 where the organic acid is a saturated or non-saturated carboxylic acid, preferably an $\alpha$-, $\beta$-, or y-hydroxy acid, a polyhydroxy acid, a dicarboxylic acid, a tricarboxylic acid, a phenolic carboxylic acid, a cyclic carboxylic acid or a hetero-cyclic carboxylic acid.

6. The diagnostic composition according to claim 5 where the carboxylic acid is a fruit acid, preferably malic acid, mandelic acid, tartaric acid, $\alpha$-hydroxy caprylic acid, oxalic acid, glycolic acid, lactic acid, citric acid, salicylic acid, gluconic acid, saccharic acid, fumaric acid, or stereoisomers of these acids, or where the carboxylic acid is a fatty acid, e.g. stearic acid, oleic acid, arachidonic acid, or a stereoisomer thereof.

7. The diagnostic reagent of one or more of the preceding claims, which contains at least two calcium salts of two different carboxylic acids, e.g. calcium lactate, calcium gluconate, calcium saccharate, calcium lactobionate, calcium ascorbate, calcium pantothenate or calcium glycolate.

8. The diagnostic reagent of claim 7, where the two calcium salts are present in an approximately equimolar ratio and preferably are calcium lacto gluconate, calcium lacto lactobionate, calcium lacto ascorbate, calcium lacto pantothenate, calcium ascorbate lactobionate (CALB), calcium lactobionate pantothenate (CLBP),and calcium ascorbate gluconate (CAG), most preferred calcium lacto gluconate.

9. The diagnostic reagent of one or more of the preceding claims, further comprising an inorganic $Ca^{2+}$ salt, preferably $CaCl_2$.

10. The diagnostic reagent of one or more of the preceding claims, wherein the reagent is in lyophilized form.

11. The diagnostic reagent of one or more of the preceding claims, wherein the glass transition temperature of the maximally frozen system Tg' of the resulting compound lies preferably above -30°C, further preferably above -20°C and most preferably above -15°C.

12. The diagnostic reagent of one or more of the preceding claims, wherein the compound resulting from a mixture of two or more organic $Ca^{2+}$ salts that interact on the molecular level in a manner that at least partially prevents crystallization of the resulting compound has a glass transition temperature Tg of at least 55°C, preferably at least 75°C, further preferably at least 80°C and particularly preferably at least 85°C.

13. A diagnostic composition or kit-of-parts comprising the diagnostic reagent of one or more of claims 1-12.

14. Use of a diagnostic reagent of one or more of claims 1-12 for recalcification in in vitro coagulation measurements of a blood sample, wherein the blood sample preferably is selected from whole blood or blood plasma and was prepared to prevent clotting activity by $Ca^{2+}$ complexing or $Ca^{2+}$ removal.

15. The use of claim 14, wherein $Ca^{2+}$ complexing was achieved by using sample tubes containing a $Ca^{2+}$ complexing agent, e.g. sodium citrate or sodium EDTA.

16. An in vitro method for measuring the coagulation of a blood sample comprising

   a) providing a blood sample from a patient,
   b) complexing $Ca^{2+}$ in the blood sample by a complexing agent, preferably sodium EDTA or sodium citrate,
   c) recalcifying the blood sample, particularly without changing the coagulation properties of the sample, by adding a suitable amount of a diagnostic reagent of one or more of claims 1-12,
   d) optionally adding further coagulation activators, coagulation inhibitors, or other substances, and
   d) performing the coagulation measurement.

17. The in vitro method of claim 16, wherein the coagulation measurement includes the activated partial thromboplastin time (aPTT), prothrombin time (PT), and/or viscoelastic tests like thromboelastometry/thromboelastography.

Figure 1

Figure 2

Figure 3

Figure 4

(a)

(b)

Figure 5

heat flow
dH/dt
[mW]

CLG 6.7200mg

Glass transition
Onset          78.70 °C
Midpoint       87.23 °C
Delta Cp       1.021 Jg^-1K^-1

2mW

30  35  40  45  50  55  60  65  70  75  80  85  90  95  100  105  110  115 °C

Figure 6

20μm

Figure 7

Figure 8a

8b

8c

8d

8e

8f

8g

Figure 9

(a)

(b)

(c)

Figure 10

Figure 11

(a)

(b)

Figure 12
(a)

(b)

(c)

Figure 13

CLG ME solution (12.5% (m/V)) 31.7100mg

**Glass transition**
**Onset** -12.64°C
**Midpoint** -11.74°C
**Delta Cp** 0.945 Jg^-1K^-1

5 mW

-18   -16   -14   -12   -10   -8   -6   -4   -2   0 °C

Figure 14

Europäisches
Patentamt

European
Patent Office

Office européen
des brevets

## EUROPEAN SEARCH REPORT

Application Number

EP 13 19 6975

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP H09 61433 A (TOKUYAMA CORP) 7 March 1997 (1997-03-07) * Pargraphes : [0008], [0010],[0011], [0014] * | 1-17 | INV. G01N33/86 |
| X | US 6 451 610 B1 (GORMAN ANNE JESSICA [US] ET AL) 17 September 2002 (2002-09-17) * abstract * * column 6, line 61 - line 64 * * column 8, line 39 - line 44 * | 1-17 | |
| A | WO 2008/093216 A1 (DSM IP ASSETS BV [NL]; CALATZIS ANDREAS [DE]; GLAUNER MARTIN [DE]; SCH) 7 August 2008 (2008-08-07) * the whole document * | 1-17 | |
| A | ATAULLAKHANOV F I ET AL: "Calcium threshold in human plasma clotting kinetics", THROMBOSIS RESEARCH, TARRYTOWN, NY, US, vol. 75, no. 4, 15 August 1994 (1994-08-15), pages 383-394, XP026404934, ISSN: 0049-3848 [retrieved on 1994-08-15] * the whole document * | 1-17 | TECHNICAL FIELDS SEARCHED (IPC) G01N |
| A | RAND M D ET AL: "BLOOD CLOTTING IN MINIMALLY ALTERED WHOLE BLOOD", BLOOD, AMERICAN SOCIETY OF HEMATOLOGY, US, vol. 88, no. 9, 1 November 1996 (1996-11-01), pages 3422-3445, XP000999799, ISSN: 0006-4971 * the whole document * | 1-17 | |

The present search report has been drawn up for all claims

| Place of search | Date of completion of the search | Examiner |
|---|---|---|
| Munich | 9 May 2014 | Jacques, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**                    EP 13 19 6975

This annex lists the patent family members relating to the patent documents cited in  the above-mentioned European search report.
The members are as contained in the European Patent Office EDP file on
The European Patent Office is in no way liable for these particulars which are merely given for the purpose of information.

09-05-2014

| Patent document cited in search report | | Publication date | Patent family member(s) | | Publication date |
|---|---|---|---|---|---|
| JP H0961433 | A | 07-03-1997 | NONE | | |
| US 6451610 | B1 | 17-09-2002 | NONE | | |
| WO 2008093216 | A1 | 07-08-2008 | CN | 101578521 A | 11-11-2009 |
| | | | EP | 2111556 A1 | 28-10-2009 |
| | | | JP | 2010518371 A | 27-05-2010 |
| | | | US | 2010190193 A1 | 29-07-2010 |
| | | | WO | 2008093216 A1 | 07-08-2008 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2202517 A1
- EP 2553470 A
- EP 0972203 A

### Non-patent literature cited in the description

- **ATAULLAKHANOV, F.I. et al.** Calcium threshold in human plasma clotting kinetics. *Thrombosis Research,* 1994, vol. 75 (4), 383-394
- **JAMES, M.F.M. ; A.M. ROCHE.** Dose-response relationship between plasma ionized calcium concentration and thrombelastography. *Journal of Cardiothoracic and Vascular Anesthesia,* 2004, vol. 18 (5), 581-586
- **RAND, M.D. et al.** Blood clotting in minimally altered whole blood. *Blood,* 1996, vol. 88 (9), 3432
- **ROCHE, A.M. et al.** Citrated Blood Does Not Reliably Reflect Fresh Whole Blood Coagulability in Trials of In Vitro Hemodilution. *Anesthesia & Analgesia,* 2003, vol. 96 (1), 58-61
- Römpp Chemie Lexikon Cm - G. 1990, 1448-1449
- **PIKAL, M. J. ; S. SHAH.** The collapse temperature in freeze drying: Dependence on measurement methodology and rate of water removal from the glassy phase. *International Journal of Pharmaceutics,* 1990, vol. 62 (2-3), 165-186
- **CHANG, B.S. ; C.S. RANDALL.** Use of subambient thermal analysis to optimize protein lyophilization. *Cryobiology,* 1992, vol. 29 (5), 632-656
- **INAGAKI, M.** Some Aspects on the Optical Resolution by Preferential Crystallization based on Phase Equilibrium. *Chemical and Pharmaceutical Bulletin,* 1977, vol. 25 (10), 2497-2503
- **FOX , T. G.** Influence of diluent and of copolymer composition on the glass temperature of a polymer system. *Bull. Am. Phys. Soc.,* 1956, 123
- **GORDON, M. ; J. S. TAYLOR.** Ideal copolymers and the second-order transitions of synthetic rubbers. i. non-crystalline copolymers. *Journal of Applied Chemistry,* vol. 2 (9), 493-500
- **HANCOCK, B. C. ; G. ZOGRAFI.** Characteristics and significance of the amorphous state in pharmaceutical systems. *Journal of pharmaceutical sciences,* 1997, vol. 86 (1), 1-12
- **MACFARLANE, D. R. ; C. A. ANGELL.** Glass transition for amorphous solid water. *The Journal of Physical Chemistry,* 1984, vol. 88 (4), 759-762
- **FRANKS, F.** Freeze-drying of bioproducts: putting principles into practice. *European Journal of Pharmaceutics and Biopharmaceutics,* 1997, vol. 45 (1998), 221-229